(19) Europäisches Patentamt
European Patent Office
Office européen des brevets

(11) **EP 3 897 761 B1**

(12) **EUROPEAN PATENT SPECIFICATION**

(45) Date of publication and mention
of the grant of the patent:
**10.09.2025 Bulletin 2025/37**

(21) Application number: **19839135.1**

(22) Date of filing: **19.12.2019**

(51) International Patent Classification (IPC):
**A61L 27/20** *(2006.01)*    **A61L 27/24** *(2006.01)*
**A61L 27/26** *(2006.01)*    **A61L 27/38** *(2006.01)*
**A61L 27/52** *(2006.01)*    **A61L 26/00** *(2006.01)*

(52) Cooperative Patent Classification (CPC):
(C-Sets available)
**A61L 27/3834; A61L 26/0023; A61L 26/0033;
A61L 26/0052; A61L 26/0057; A61L 26/0076;
A61L 26/008; A61L 27/20; A61L 27/24;
A61L 27/26; A61L 27/52;** A61L 2300/64    (Cont.)

(86) International application number:
**PCT/IB2019/061072**

(87) International publication number:
**WO 2020/128932 (25.06.2020 Gazette 2020/26)**

(54) **BIOMATERIAL AND USE THEREOF IN THE TREATMENT OF LUNG PATHOLOGIES**

BIOMATERIAL UND SEINE VERWENDUNG ZUR BEHANDLUNG VON LUNGENERKRANKUNGEN

BIOMATÉRIAU ET SON UTILISATION DANS LE TRAITEMENT DE PATHOLOGIES PULMONAIRES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **21.12.2018 IT 201800020722**

(43) Date of publication of application:
**27.10.2021 Bulletin 2021/43**

(73) Proprietors:
• **BORZACCHIELLO, Assunta**
  **80135 Napoli (IT)**
• **Messina, Francesco**
  **80123 Napoli (IT)**

(72) Inventors:
• **DELLA SALA, Francesca**
  **80126 Napoli (IT)**
• **BORZACCHIELLO, Assunta**
  **80135 Napoli (IT)**
• **MESSINA, Francesco**
  **80123 Napoli (IT)**

(74) Representative: **Botti & Ferrari S.p.A.**
**Via Cappellini, 11**
**20124 Milano (IT)**

(56) References cited:
**EP-A1- 2 554 176        WO-A1-03/015802
WO-A1-2015/023720    WO-A1-2015/073786
WO-A1-2017/164467    WO-A2-2011/047345
WO-A2-2014/127232    WO-A2-2018/085516
US-A1- 2014 227 235**

• **SHEEHAN K M ET AL: "Hyaluronic acid of high
molecular weight inhibits proliferation and
induces cell death in U937 macrophage cells",
LIFE SCIENCES, PERGAMON PRESS, OXFORD,
GB, vol. 75, no. 26, 12 November 2004
(2004-11-12), pages 3087 - 3102, XP004601752,
ISSN: 0024-3205, DOI: 10.1016/J.LFS.2004.02.038**

Remarks:
The file contains technical information submitted
after the application was filed and not included in this
specification

(52) Cooperative Patent Classification (CPC): (Cont.)

C-Sets
**A61L 26/0023, C08L 5/08;**
**A61L 26/0052, C08L 5/08;**
**A61L 26/0052, C08L 89/06;**
**A61L 27/20, C08L 5/08;**
**A61L 27/26, C08L 5/08;**
**A61L 27/26, C08L 89/04**

**Description**

FIELD OF THE INVENTION

The present invention relates to the biomedical sector of the treatment of lung diseases and related symptoms. In particular, the present invention provides compositions and methods based on the use of selected polymeric biomaterials, in combination with stem cells and/or their secretome, capable of synergistically improving the development, regeneration and repair of chronic lung injuries and related symptoms.

BACKGROUND TECHNIQUE

Chronic lung diseases including chronic obstructive pulmonary disease (COPD), idiopathic pulmonary fibrosis (IPF), asthma and pulmonary bronchodysplasia (BPD) of the premature infant present, as common denominator, lung injuries that cannot be resolved but lead to an aberrant tissue repair, thus causing an arrest of alveolar growth in BPD or alveolar destruction in COPD.

In particular, asthma and COPD are common disorders affecting 1 in 12 people worldwide. Asthma and COPD are chronic inflammatory diseases characterized by airway obstruction that are reversible in asthma and often irreversible in COPD. Another important feature of COPD, and occasionally also of severe asthma, is emphysema which involves the destruction of alveolar tissue, with a consequent reduction in the oxygen exchange. The airways are made up of a pseudostratified epithelium that includes basal, ciliated and secretory cells. Furthermore, in both asthma and COPD, the barrier function of the epithelium has decreased, since intercellular junction proteins have been destroyed [Postma et al, The New Engl Jour of med 2015, Slats et al Therap advan in resp disease 2016, Gohy et al J of the British Society for Aller and Clin Immuno 2016]. Other shared features of asthma and COPD include calyx cell metaplasia with increased mucus production, alteration of inflammatory reactions, decreased expression and activity of the antimicrobial peptide, and alteration of basal function that can lead to defective responses after the lesion [Mertens et al Pulm Pharmacol Ther 2017]. BPD is a chronic lung disease of the preterm infant, it remains one of the main causes of morbidity and mortality, and moreover the percentage of premature births is constantly increasing. The risk of developing BPD is inversely related to gestational age and birth weight. Premature new-borns (24-28 weeks of gestation) are born during the canalicular or saccular phases of lung development. The lung structure, characterized by a thickened air space and walls with a lack of surfactant, is not able to provide sufficient ventilation and gas exchange. For this reason, mechanical ventilation and high oxygen concentrations are often required for birth. Thus, barotrauma induced by mechanical ventilation as well as oxygen toxicity and inflammation are factors that contribute for the most part to morphological and functional damage in the immature lung. Historically, oxygen toxicity and damage induced by mechanical ventilation were the prerequisites for the attribution of BPD in premature babies, the so-called "old BPD". Currently, the increase in the survival rate of infants of ever lower gestational age has led to the definition of a pathology labelled as "new BPD", for which the histological results of the lungs have shown an altered development of the alveoli, in lower numbers and larger size, and a dysmorphic growth at the microvascular level, together with a lower degree of inflammation and fibrosis than the "old BPD".

Currently, an effective curative therapy is not available. Based on BPD severity definition (including infants with mild BPD) 68% of premature infants born with a gestational age (GA) ≤28-29 weeks develops BPD. In addition, severe BPD is associated with impaired neuronal development, pulmonary hypertension and death [Bhandari et al Pediatrics 2009, Chao CM et al Front Med 2015]. Recent insights into stem cell biology have generated enthusiasm about the potential of stem cells to regenerate damaged organs [Pierro et al Thorax 2013, Beers et al J Clin Invest 2016]. Mesenchymal stem cells (MSCs) have attracted much attention because of their ease of isolation, multilinear differentiation potential and immunomodulatory properties. Adult bone marrow MSCs prevent lung injury in various experimental models of lung disease, including those on BPD. MSCs can be isolated from several sources, including cord blood or umbilical cord, two sources of neonatal cells that have unique advantages compared to adult MSC [Pierro et al Thorax 2013].

The fate of stem cells in vivo is mainly regulated by the microenvironment [Pierro M, Thébaud B et al Thorax 2013]. Epithelial-mesenchymal interaction has been shown to be critical in normal lung development. Fibroblasts are the main type of cells present in the lung microenvironment that can induce type II alveolar epithelial cells to mature and express the SPC gene.

MSCs offer a variety of potentially beneficial mechanisms for the treatment of lung diseases by reducing lung inflammation, fibrillation, oxidative stress and apoptosis. To assess their potential in preventing BPD, the effectiveness of MSC therapy on lung immaturity has yet to be determined. Their mode of action seems to be mainly attributed to the paracrine mechanisms that favor alterations of the niche/tissue environment, with the release of pro-angiogenic and anti-apoptotic factors, production of extracellular matrix (ECM), exchange of organelles such as mitochondria through micro vesicles, immunomodulatory activities and anti-inflammatory properties [Laube et al Int J Biochem Cell Biol 2016].

Preliminary results of the use of MSCs are reported in the first clinical study of MSCs on infants with BPD in 2014 called PNEUMOSTEM. This product is made up of cell cultures of Mesenchymal allogeneic ex-vivo stem cells (hUCB-MSCs) derived from human umbilical cord blood, [Chang YS et al J Pediatr 2014], administered in a "dose escalation" study (1-2 x $10^7$ cells per kilogram) through an endotracheal tube in extremely premature babies born at 27 weeks of gestation for a period of 5 to 14 days. Compared to historical controls at the same ages, treated infants developed less severe BPD and had reduced levels of inflammation and no treatment-related adverse effects were observed. It is currently underway the registration in the United States (clinicaltrials.gov; NCT02381366) of an open-label dose escalation study for these premature babies [Nitkin CR et al Stem Cells Transl Med 2017, O'Reilly, Thébaud et al CellTissue Res 2017].

Some examples of lung injury treatments known in the patent literature, for example, are the patent documents: US 5,633,003 Cantor, US 2008/0311088 Chang et al., US 2010/0266552 Jenkin et al., US 2009/0274665 Akabutu et al. In these documents the treatments are heterogeneous, but none of them focus on compositions that allow an increase in the therapeutic efficacy of MSCs, for the treatment of lung diseases.

Patent application WO2017/164467 A1 describes a biocompatible scaffold for the cultivation of mesenchymal stem cells comprising a cell-like material, selectable within an open list of single natural polymers; the cells thus obtained have anti-inflammatory immunostimulating properties and are proposed in the treatment of a variety of pathologies, including for example those related to lungs. The patent application WO2018/085516 A2 describes compositions of non-mesenchymal lung stem cells characterized by specific markers, and a suitable pharmaceutical carrier which can be indifferently constituted by a variety of biocompatible materials. Patent application US2014/0227235 A1 describes implants based on collagen, hyaluronic acid, in particular cross-linked, and optionally stem cells, as supports for the treatment of cartilage diseases; apart from the nature of cross-linked derivative, the document does not provide information about the type of hyaluronic acid: however the type of application described, which involves the formation of implants, is compatible with the use of systems with high viscosity and presumably with high molecular weight; in the experimental part, the authors measure the differentiation capability of stem cells in a collagen-only scaffold. Patent application WO03/015802 A1 describes compositions for the treatment of cartilage joint damage based on a variety of cellular components, e.g. mesenchymal stem cells, in the presence of a variety of biocompatible polymers, e.g. hyaluronic acid, chitosan, pluronic acid, etc.

Patent applications WO2015/073786 A1 and WO2011/047345 A2 disclose a method to treat or prevent a lung disorder by administration of a population of isolated or enriched umbilical cord tissue-derived MSCs. Patent application EP 2 554 176 A1 discloses a composition comprising hyaluronic acid and mesenchymal stem cells. Patent application WO2014/127232 A2 discloses a composition comprising low molecular weight hyaluronic acid, collagen and human MSCs suitable for various applications, including the treatment of the lung. Patent application WO2015/023720 A1 discloses a method of differentiating MSCs into a lung cell, the method comprising: seeding MSCs on a biomaterial comprising an extracellular matrix comprising a mixture of fibrillar collagen and hyaluronic acid of unknown moellculr weight. The publication Life Sciences, 75(26), 2004, p-3087-3102 highlights the effect of low molecular weight hyaluronic acid on cell proliferation and survival. Patent application WO2010/114319 A2 discloses a composition comprising hyaluronic acid with MW 110 KDa and MSCs.

In chronic lung diseases, such as BPD, COPD and IPF, it has been reported that lung injury regeneration, only through MSC-based therapies, is more difficult to achieve. Since, the loss and alteration of the ECM supporting the pulmonary architecture can preclude the repair of the normal alveolar structure. Indeed, ECM is a complex entity fundamental in many biological processes and with distinct signals, which helps to guide cell adhesion, survival, proliferation and moreover the remodelling of the ECM is involved in the postnatal alveolarization process [Pittenger et al. Science 1999, Baksh et al. Stem Cells 2007, 10 Collins JJP et al. Front Med (Lausanne) 2017].

Therefore, further additional strategies combining the use of a therapy based only on MSC are necessary [Collins, Thebaud et al, Birth Defects Research (part A) 2014] to increase the effectiveness of the repair of lung damage in order to obtain a more complete regeneration of the lung tissues.

SUMMARY OF THE INVENTION

The present inventors have found new biomaterials, particularly suitable for the pulmonary route administration of mesenchymal cells, based on the use of a structured combination of selected biopolymers. These biomaterials have proved to be particularly effective in preserving and promoting the viability and differentiation of mesenchymal cells, whether they are included in the biomaterial itself or in contact with biomaterial after its formation.

A first object of the invention is therefore a biomaterial, comprising:

    (a) hyaluronic acid or a salt thereof, having a molecular weight ranging between 100 and 500 KDa;
    (b) collagen;
    (c) human mesenchymal stem cells and/or their secretome.

**EP 3 897 761 B1**

The biomaterial contains two phases, wherein at least part of component (b) is present as a separate phase from component (a), said separate phase also comprising the component (c).

A second object of the invention is the abovementioned biomaterial for use in therapy, in particular in the treatment of lung diseases. A method for the preparation of the above mentioned biomaterial, not claimed, is also described.

DESCRIPTION OF THE FIGURES

**Figure 1:** Percentage of viability of hUCMSCs after 24 h of contact with biomaterial 1 (panel a) or 2 (panel b), measured as described in Example 3.

**Figure 2:** Percentage of viability of hUCMSCs after 24 h of contact with biomaterial 3 and with different concentrations of biomaterial 1, measured as described in Example 3.

**Figure 3:** Percentage of viability of hUCMSCs after 24 h of contact with different concentrations of biomaterial 4, measured as described in Example 3.

**Figure 4:** Confocal images of differentiation in type II alveolar cells after 15 and 21 days of incubation with biomaterial 1 in System 1, as described in Example 4.

**Figure 5:** Confocal images of differentiation in type II alveolar cells after 15 and 21 days of incubation with biomaterial 1 in System 2, as described in Example 4.

**Figure 6:** Confocal images of differentiation in type II alveolar cells after 15 and 21 days of incubation with biomaterial 4 in System 3, as described in Example 4.

**Figure 7:** Effect of biomaterials on the differentiation of hUCMSCS in type II alveolar cells of Example 3.2. Quantitative analysis of the confocal images of the expression of the SPC protein in System 1 and 2 in the presence of biomaterial 1 (a) and biomaterial 3 and biomaterial 1 (b), as described in Example 4.

**Figure 8:** Mechanical spectrum of biomaterial 3, as described in Example 5.

**Figure 9:** Mechanical spectrum of biomaterial 3 and biomaterial 4 at two different concentrations of hyaluronic acid, 5 and 10 mg/ml, as described in Example 5.

**Figure 10:** Percentage of viability of the hUCMSCs after 24 h of contact with different concentrations of biomaterial 2 in System 2, measured as described in Example 3.

**Figure 11:** Quantitative expression of the surfactant SPC protein after 21 days of incubation with biomaterials 5 and 6 in System 3.

DEFINITIONS

The term "biomaterial" according to the present invention refers to a composition which is suitable to be introduced at the level of a tissue or organ of the human body and thereby to induce or assist a physiological response.

The term "polysaccharide" according to the present invention refers to biological macromolecules composed of the union of numerous monose molecules, identical or different, linked together by a covalent bond.

The term "semi-synthetic polysaccharide" refers to a polysaccharide obtained by modifying, through one or more chemical reactions, a natural polysaccharide.

The term "connective tissue protein" refers to proteins found naturally in animal connective tissues.

The terms "gelling compound", "gelling protein" or "gelling polymer" refer to a compound, protein or polymer, respectively, which produces, once dissolved in water or in an aqueous solution, a gelling solution, as defined below. Preferably, said compound, protein or polymer gives rise to a solution that can be gelled at physiological pH and temperature conditions: for example it is selected from type I collagen, albumin and pluronic copolymers.

The term "pluronic copolymer" refers to symmetrical three block copolymers comprising polyethylene oxide and polypropylene oxide in linear alternation.

The terms "mesenchymal stem cells", "mesenchymal cells", or "multipotent stromal cells (MSC)", used here as synonyms, refer to cells having a very similar appearance to fibroblasts and capable of forming colonies and replicating and differentiating, following stimulation with appropriate growth factors and conditioning culture media; for example, they can differentiate into alveolar lineage, in particular into type II alveolar cells, in the presence of the SAGM culture medium marketed by Lonza. The term "hyaluronic acid" refers to a linear polysaccharide consisting of alternating units of a repeated disaccharide, acid $\beta$-1,4-D-glucuronic-$\beta$-1,3-N-acetyl-D-glucosamine. Hyaluronic acid is also known as hyaluronan, hyaluronate or HA. The terms hyaluronan, hyaluronic acid and HA are used interchangeably in this document.

The term "molecular weight", when referred to the polymers used in the present invention, refers to the weight average molecular weight (Mw).

The term "gel" according to the present invention refers to a swollen network with a high percentage of water and

5

capable of maintaining its own shape.

The term "gelling solution" according to the present invention refers to a solution which is capable of gelling under specific conditions of pH and temperature. In the present invention, solutions which can gel under physiological pH and temperature conditions are of particular interest, in particular at pH between 7 and 7.8 and temperature between 36° and 37°C.

The term "viscous solution" refers to a solution having a viscosity of at least $1 \times 10^{-3}$ Pa s.

The term "phase" or "separate phase", referring to the present biomaterials, identifies a volumetric sub-portion of the biomaterial itself, distinguishable by its particular quali/quantitative composition; the distinction between the phases can be further highlighted by changes in physical characteristics, such as for example viscosity, density, mechanical properties, etc. The neighbouring phases are separated by an interface which can be, depending on the case, a clean border or a transition area within which the adjacent portions of the two phases interpenetrate.

The term "mesenchymal cell secretome" (synonymous with the terms "stem cell secretome"/"stromal cell secretome "), mentioned here briefly as "secretome", refers, in accordance with the knowledge of the area of expertise, to a mixture of products secreted by mesenchymal cells including paracrine factors useful for intercellular communication and development, homeostasis and tissue (re)generation; examples of these paracrine factors are: brain-derived neurotrophic factor (BDNF), nerve growth factor (NGF), insulin growth factor 1 (IGF-1), hepatocyte growth factor (HGF), vascula **endothelial** growth factor (VEGF), transforming growth factor β (TGF-β), glial-derived neurotrophic factor (GDNF), fibroblast growth factor 2 (FGF-2), stem cell factor (SCF) granulocyte colony stimulating factor (GCSF), stromal cell-derived factor (SDF), etc. The secretome may further comprise an insoluble fraction consisting of extracellular vesicles (typically exosomes, microvescicles, membrane particles, peptides, small proteins such as cytokines, etc.).

## DETAILED DESCRIPTION OF THE INVENTION

The present inventors have identified a new biomaterial particularly suitable for the treatment of lung diseases, which allows to overcome the limits found using only mesenchymal cells.

A first object of the invention is therefore a biomaterial, comprising:

(a) hyaluronic acid or salt thereof, having a molecular weight ranging between 100 and 500 KDa;
(b) collagen;
(c) human mesenchymal cells and/or their secretome.

The biomaterial contains two phases, wherein at least part of component (b) is present as a separate phase from component (a), said separate phase also comprising the component (c).

The biomaterial is preferably an aqueous gel or an aqueous viscous solution. The abovementioned components (a) - (c) can be, depending on their solubility, dissolved or dispersed within the biomaterial.

In all the forms and embodiments of the invention, the biomaterial has a physiological pH, in particular compatible with the pulmonary environment, more particularly between 7 and 7.8.

In all forms and embodiments of the invention, the biomaterial is preferably in the form of a gel or a viscous solution, having at least one aqueous liquid phase.

In all the forms and embodiments of the invention, said hyaluronic acid or salt thereof is preferably contained in the aforementioned biomaterial at a concentration by weight of 0.001 to 20%, more preferably of 0.001 to 10%, even more preferably of 0.005 to 10%, referred to the weight of the biomaterial not considering component (c) (cells and/or their secretome).

In all forms and embodiments of the invention, the biomaterial has a viscosity (meant as dynamic viscosity, measured at a temperature of 25°C in a steady - state shear flow at shear rate equal to 10 sec$^{-1}$, preferably between 0.001 and 100 Pa s (for example between 0.001 and 10 Pa s; or between 0.004 and 10 Pa s; or between 0.001 and 1 Pa s; or between 0.004 and 0.1 Pa s; or any other interval between these limits). The viscosity of the biomaterial can be chosen according to the used route of administration, and obtained by changing the concentration and molecular weight of the components (a) and/or (b); in particular, for component (a) the viscosity increases with the increase of both the concentration and the molecular weight (M). For example, for diluted polymeric systems the viscosity increases as $M^1$, while for concentrated polymeric systems it increases as $M^{3.4}$. The abovementioned viscosity ranges are also valid in the case of biphasic biomaterials: in this case the two phases can have different viscosities, each of which being within the abovementioned ranges. Low molecular weight hyaluronic acid or salt thereof (component (a)), preferably together with collagen in accordance with the invention, possesses optimal characteristics of stimulating the viability and the differentiation of human mesenchymal cells. In a particularly preferred embodiment, the aforementioned hyaluronic acid can be used in the form of salt thereof, preferably a sodium salt. To achieve the high viability of mesenchymal cells and fulfil the objectives of the present invention, it is essential that the molecular weight of hyaluronic acid is not higher than 500 KDa, and generally between 100 and 500 KDa. Indeed, as shown in the experimental part, the use of hyaluronic acid with molecular weights higher than 500 KDa leads to the loss of the ability of the biomaterial to stimulate the viability of the mesenchymal cells. Preferred sub-ranges for low molecular weight hyaluronic acid are, for example: between 100 and 200 KDa, between 100 and 300 KDa, between 100 and 400 KDa, between 100 and 500 KDa; or between 120 and 200 KDa, between 120 and 300 KDa, between 120 and 400 KDa, between 120 and 500 KDa; or between 160 and 200 KDa, between 160 and 300 KDa, between 160 and 400 KDa, between 160 and 500 KDa; most preferred is the range between 160 and 240 KDa and, internally, the value of approximately (i.e. $\pm$ 20) 200 KDa, e.g. 200 KDa.

Collagen (component (b)), together with low molecular weight hyaluronic acid in accordance with the invention, has optimal characteristics in stimulating the viability and the differentiation of the mesenchymal cells; in particular, as the experimental part shows, it significantly amplifies the aforementioned effects on mesenchymal cells obtained from low molecular weight hyaluronic acid: for this reason, the presence of collagen in addition to low molecular weight hyaluronic acid, albeit not restrictive for the purposes of the invention, is particularly preferred. In addition, collagen has gelling properties. Type I or type III collagen is preferably used, even more preferably type I collagen. These proteins are physiologically present in high quantities in the interstitial connective tissue of the lungs, where they form band patterned fibrils from 500 to 1000 $\mu$m.

Type I collagen has interesting gelling properties, as it gives rise to solutions that gel at body temperature (37°C) and at a pH between 7 and 7.8. This allows to obtain a biomaterial with particularly advantageous characteristics, because it is presented in the form of a viscous solution before the administration and increases the viscosity by gelling once administered to the patient. Preferably the mentioned collagen, e.g. type I or type III collagen, is present in the biomaterial at a concentration by weight of 0.01 to 1%, preferably from 0.1 to 1%, more preferably from 0.1 to 0.5% referred to the total weight of the biomaterial, not considering the component (c) (mesenchymal cells and/or their secretome).

Mesenchymal stem cells (component (c)) used in the present biomaterials are typically of human origin. They can preferably be obtained from the umbilical cord, circulating blood, bone, adipose tissue or placenta through procedures well known to those with expertise in this field [Han YF et al Cytotechnology 2013, Smith JR et al Curr Protoc Stem Cell Biol 2017]. Particularly preferred are human mesenchymal cells derived from umbilical cord (also referred to as hUCMSCs). Indeed, these adult stem cells possesses a significant self-renewal capacity and multilinear differentiation ability. The cells obtained from the umbilical cord can be used from the first extraction of the tissue (primary culture) up to the sixth passage (secondary culture), where for passage we mean that the cells of the primary culture are removed from the plate and cultured at a lower density in another plate. hUCMSCs can also be obtained directly from commercial sources, for example "Umbilical Cord derived mesenchymal cells; Normal, Human" marketed by ATCC PCS-500-010.

Mesenchymal stem cells are dispersed in the biomaterial preferably at a density between $6 \times 10^3$ and $10 \times 10^3$ cells/ml.

In an advantageous embodiment of the invention, in addition or in alternative to human mesenchymal cells, the biomaterial object of the invention may contain secretome of human mesenchymal cells. Indeed, it has been observed that the presence of the secretome further increases the differentiation capability of the mesenchymal cells present in the biomaterials or come in contact with it. The secretome used in the invention is preferably derived from human adipose mesenchymal cells. Preferably, it is in lyophilized form and as such added in the preparation process of the present biomaterials. The concentration by weight of secretome is preferably between 0.5% and 20% referred to the total weight of the biomaterial, not considering the component (c) (mesenchymal cells and/or secretome).

In addition to the aforementioned fundamental components, the present biomaterial further comprises one or more nutrients for the mesenchymal stem cells, preferably dissolved in the aqueous liquid phase. Preferably, said nutrients are selected from L-glutamine, red phenol, sodium pyruvate and sodium bicarbonate.

Preferably, the aforementioned biomaterial further comprises preferably electrolytes and pH regulators which make the osmolarity and pH characteristics of the biomaterial acceptable for administration in the lung.

The biomaterial may further comprise a culture medium containing nutrients such as the culture medium DMEM, SAGM, McCoys, CMR GMEM, HM, INDM, LM, RPMI, and/or one or more substances that stimulate cell differentiation of mesenchymal cells in alveolar cells and/or one or more antibiotics. Preferably, the said substances which stimulate cell differentiation are selected from bovine serum albumin (BSA), preferably at the concentration of 0.5 mg/ml, insulin, preferably at the concentration of 5 mg/ml, transferrin, preferably at the concentration of 10 mg/ml, bovine pituitary extract, preferably at the concentration of 30 mg/ml, adrenaline (epinephrine), preferably at the concentration of 0.5 mg/ml, triiodothyronine, preferably at the concentration of 6.5 ng/ml, retinoic acid, preferably at the concentration of 0.1 ng/ml, cortisol, preferably at the concentration of 0.5 ml/ml, human epidermal growth factor (EGF), preferably at concentration of 0.5 ng/ml, fetal bovine serum (FBS), preferably at a concentration of 5%.

Preferably, the aforementioned antibiotics are selected from penicillin G, streptomycin or gentamicin sulfate and/or one or more antifungal agents, preferably selected from amphotericin B and nystatin. A method for the preparation of the aforementioned biomaterials is described; in its most general form, it includes putting them in contact with each other:

(a) hyaluronic acid or salt thereof, having a molecular weight ranging between 100 and 500 KDa,

(b) collagen,

(c) human mesenchymal cells and/or their secretome.

Said method can be adapted to the preparation of biomaterials in accordance with the structural arrangement envisaged by the present invention.

Accordingly, a homogeneous aqueous solution of component (b) (collagen) is prepared. The mesenchymal cells and/or their secretome are then homogeneously added and dispersed to the aqueous solution, optionally sterilized by filtration; the method may include a further stage wherein the resulting dispersion is subject to gelification. The resulting material is then put in contact without mixing, for example by deposition/stratification, on a substrate in the form of a viscous solution comprising nutrients and component (a) (hyaluronic acid or salt thereof).

Optional components aforementioned above (nutrients, electrolytes, etc.) can be incorporated indifferently into one or more of the phases mentioned; preferably they are present, totally or mainly, in the phase in which the mesenchymal cells are present.

As demonstrated in the experimental part, the biomaterial according to the invention stimulates the proliferation and differentiation of mesenchymal cells, in particular of human origin, in particular in alveolar cells, being thus suitable to produce, after administration to a patient who needs it, a therapeutic effect on chronic lung injuries. The aforementioned effects are obtained on mesenchymal cells in general, that is, not only on the mesenchymal cells contained in the biomaterial, but also on the mesenchymal cells subsequently come in contact with it, for example endogenous mesenchymal cells of a patient to whom the biomaterial of the invention was administered, in all its variants.

A further object of the invention is therefore a biomaterial as defined above for use in therapy, in particular for use in the treatment of lung diseases.

A further object of the invention is the use of a biomaterial as defined above in the production of a medicament, in particular for the treatment of lung diseases.

Preferably, said lung pathologies are chosen among chronic obstructive pulmonary disease (COPD), asthma and lung bronchodysplasia (BPD) of the premature infant.

The biomaterial according to the invention can be administered through any route of administration that allows its transport at the level of the pulmonary alveoli.

According to a preferred application, the biomaterial has a viscosity comprised between $1 \times 10^{-3}$ Pa s and 1 Pa s, preferably between $4 \times 10^{-3}$ Pa s and $1 \times 10^{-1}$ Pa s and it is administered intratracheally via oro-tracheal or nasal-tracheal ventilation cannulas. In particular, this route of administration is suitable for viscous solutions having the aforementioned viscosity or for viscous solutions gelling at conditions of pH and physiological temperature, and which therefore gel at the level of the lung tissues at body temperature. According to a further preferred application, the biomaterial of the present invention can be administered by aerosolization. The biomaterial has a viscosity between $1 \times 10^{-3}$ Pa s and $1 \times 10^{1}$ Pa s, preferably between $4 \times 10^{-3}$ Pa s and $5 \times 10^{-2}$.

Once administered, the biomaterial acts at the lung tissue level with a synergistic effect on alveolar regeneration as a result of the damage caused by inflammatory processes and/or immature and altered alveolar structure.

The present invention will be illustrated below in a non-limitative manner by the following examples.

EXPERIMENTAL PART

The preparation of the samples of biomaterials having the following composition in hyaluronic acid and/or collagen is described:

Table 1: Hyaluronic acid and collagen composition of biomaterials 1 to 4.

| Biomaterials | Biomaterial 1 (reference) | Biomaterial 2 (reference) | Biomaterial 3 (reference) | Biomaterial 4 (invention) |
|---|---|---|---|---|
| HA* 200 KDa | 1 mg/ml; 2.5 mg/ml; 5 mg/ml; 7,5 mg/ml e 10 mg/ml | | | 1 mg/ml; 5 mg/ml; e 10 mg/ml |
| HA* 1435 KDa | | 1 mg/ml; 2.5 mg/ml; 5 mg/ml e 10 mg/ml | - | |
| Collagen | | | 2.4 mg/ml | 2.4 mg/ml |
| (* HA: hyaluronic acid) | | | | |

## Example 1: Preparation of bioprecursors

C) The preparation of the biopolymer part of the present biomaterials, that is the respective precursor materials before the addition of the mesenchymal and/or secretome cells, is described*A) Bioprecursors 1p and 2p (precursors of biomaterials 1 and 2)*

HA solutions with the weight concentrations reported in table 1 were prepared, using HA having a molecular weight of 200 kDa (1p) or 1435 kDa (2p). In particular, samples, in the form of solutions in the culture medium suitable for the experiment to carry out, were prepared, at the following concentrations of hyaluronic acid: 1 mg/ml; 2.5 mg/ml; 5 mg/ml; 7.5 mg/ml; and 10 mg/ml for the biomaterial 1p and 1 mg/ml; 2.5 mg/ml; 5 mg/ml; and 10 mg/ml for the 2p biomaterial.

To carry out the cell viability experiments, a suitable amount of hyaluronic acid in the form of sodium salt was weighed and dissolved in a suitable volume of DMEM (Microgem Italia L0064-500) then stirring for 3 hours in order to guarantee the homogeneity of the solution. In detail, to carry out the differentiation experiments, a suitable amount of hyaluronic acid in the form of sodium salt was weighed and it was dissolved in a suitable volume of SAGM (Lonza C-4124) and then kept under stirring for 3 hours in order to guarantee the homogeneity of the solution. The solutions obtained from the previous procedure were sterilized by filtration using 0.22 micron filters.

### *B) Bioprecursor 3p (precursor of biomaterial 3)*

8 ml of a solution of type I Collagen of bovine origin (Sigma Aldrich cat.n. fD 3510-100) was added at a temperature of 4°C to 2 ml of a filtered solution of Dulbecco phosphate buffer 10X (D -PBS, Gibco), obtaining a final collagen concentration of 2.4 mg/ml.

The solution thus obtained is used to give rise to a collagen gel, adding drops of NaOH and/or HCl to adjust the pH to 7.2 and placing the solution in an incubator at the temperature of 37°C for 40 minutes to allow the fibrillogenesis process.

### *C) Bioprecursor 4p (precursor of biomaterial 4)*

Various solutions have been prepared, containing collagen at a concentration of 2.4 mg/ml and hyaluronic acid with a molecular weight of 200 kDa at different final concentrations: 1 mg/ml; 5 mg/ml; and 10 mg/ml. In detail, 100 mg of hyaluronic acid was dissolved in a specific container in 10 ml of D-PBS solution to prepare a stock solution, so as to obtain a solution with a hyaluronic acid concentration of 10 mg/ml. The solution thus obtained was filtered and diluted with suitable volumes according to a 2X dilution factor to obtain the concentration of 5 mg/ml and according to a 10X dilution factor to obtain the solution at 1 mg/ml concentration. The solutions thus obtained are added, at a temperature of 4°C, to the collagen solution prepared as described in Example 3, to obtain final concentrations of hyaluronic acid of 1 mg/ml; 5 mg/ml; and 10 mg/ml.

The solution thus obtained is used to give rise to a collagen gel, adding few drops of NaOH and/or HCl to adjust the pH to 7.2 and placing the solution in an incubator at a temperature of 37°C for 40 minutes to allow the fibrillogenesis process.

## Example 2: Extraction and culture of hUCMSCs

The hUCMSCs were extracted from the umbilical cords according to the compliant methods [Han YF et al Cytotechnology 2013] and can be cryopreserved according to the compliant methods [Smith JR et al Curr Protoc Stem Cell Biol 2017].

In detail, the umbilical cord tissue was removed from a sterile 0.9% sodium chloride solution containing Penicillin (200 units/mL) -Streptomycin (200 $\mu$g/ml) in a laminar flow hood and washed twice with a phosphate buffer solution (PBS). The umbilical cord, about 10 cm long, was cut into three equal parts. Each piece was in turn cut into small pieces of about 1 cm in length, which were then washed thoroughly to remove blood and blood clots, umbilical arteries, veins and umbilical cord adventitia were removed to obtain Wharton jelly. Then, the pieces containing Whar'on's jelly were sectioned into small pieces of about 1 mm$^3$ and used to isolate and cultivate primary hUCMSCs. Pieces of tissue, correctly cut, were placed in plates for tissue cultures of 10 cm and spaced out of 0.5 cm. Complete medium (Dulbe'co's modified Ea'le's medium (DMEM)) was added, implemented with 10% (vol/vol) of fetal bovine serum (FBS), penicillin (100 units/ml), and streptomycin (100 $\mu$g/ml), the tissues were grown in an incubator with 5% $CO_2$ and saturated humidity at 37°C. 5 ml of complete medium was added 4 hours later for an extended culture period, and the media were changed every 3-4 days. The adhesion and growth of primary cells were observed using an inverted phase contrast microscope.

For storage, the prepared hUCMSCs were suspended at the density of 1 x 10$^6$ cells per ml in a medium for cryopreservation, consisting of 10% to 20% fetal bovine serum (FBS) and 5-10% dimethylsulfoxide (DMSO). The cell suspension was stored in criovials in a device that reduces the temperature by about 1°C per minute at controlled speed, so as to keep them at -80°C. They were then transferred to a liquid nitrogen tank, kept at -150°C.

## Example 3: Effect of biomaterials on the viability of hUCMSCs

To assess the viability of the cells prepared according to the procedure described in Example 2 when put in contact with biomaterials 1-4, the Alamar Blue (Invitrogen) test was performed.

The Alamar Blue test was performed by adding the Alamar Blue (AB) reagent to the cells in contact with biomaterials 1-4 and incubating at 37°C for 4 hours.

The absorbance of the samples was measured using a spectrophotometer plate reader (Multilabel Counter, 1420 Victor, Perkin Elmer) at 570 nm and 600 nm. AB is a dye that incorporates an oxidation-reduction indicator that changes colour in response to the chemical reduction of the growth medium, resulting from cell viability. The results are expressed as a percentage difference between the treated samples and controls and are calculated with the following formula:

$$\% reduction = \frac{(O_2 \times A_1) - (O_1 \times A_2)}{(O_2 \times P_1) - (O_1 \times P_2)} \times 100$$

Where O1 is the molar extinction coefficieE(E) of AB oxidized at 570 nm; O2 is AB oxidized at 600 nm; A1 is the absorbance of the test wells at 570 nm; A2 is the absorbance of the test wells at 600 nm; P1 is the absorbance of the positive control well at 570 nm; P2 is the absorbance of the positive control well at 600 nm.

### Biomaterials 1 and 2

HUCMSCs cells prepared as described in Example 2 were seeded on the flat surface of petri dishes and incubated for 24 hr with biomaterials 1 and 2 containing DMEM and different concentrations of hyaluronic acid prepared in Example 1A or with DMEM not supplemented with hyaluronic acid (control).

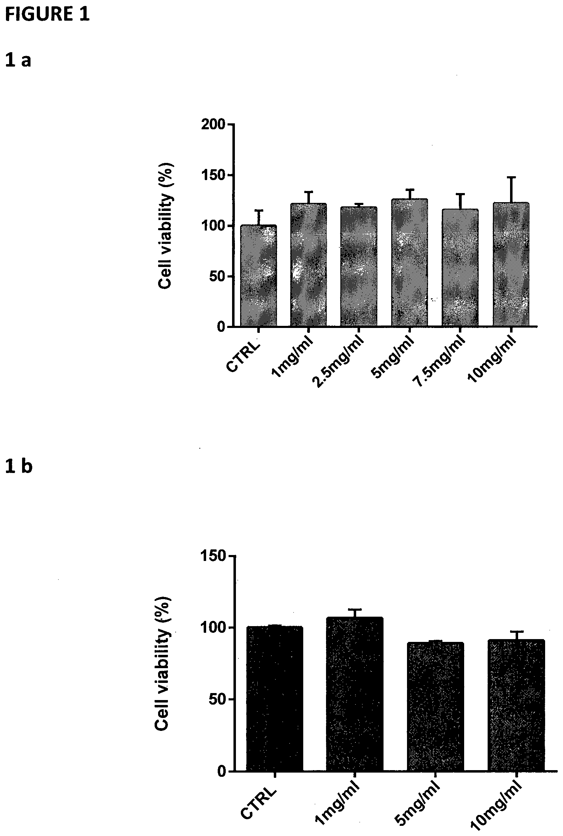

After this time the Alamar blue reagent is added to the samples and incubated at 37°C for 4 hr. The results are shown in figure 1 (panel 1a for biomaterial 1 and panel 1b for biomaterial 2) and expressed in terms of percentage of viability calculated as reported above, at the different concentrations of hyaluronic acid in biomaterials 1 and 2.

From figure 1a it can be seen that biomaterial 1, containing low molecular weight HA, has a stimulating effect on cell viability compared to the control; indeed, at all analysed concentrations, it is possible to observe an increase of the viability of hUCMSCs cells over 100%; conversely, in figure 1b, biomaterial 2 containing high molecular weight HA did not show significant effects on cell viability compared to the control.

### Biomaterial 4 (System 2)

The hUCMSCs were combined with the collagen solution described in Example 1b at pH 7.2 and the suspension thus obtained was put at 37°C for an adequate time to allow the fibrillogenesis in the presence of the cells and therefore to obtain a three-dimensional composite gel consisting of collagen and hUCMSCs.

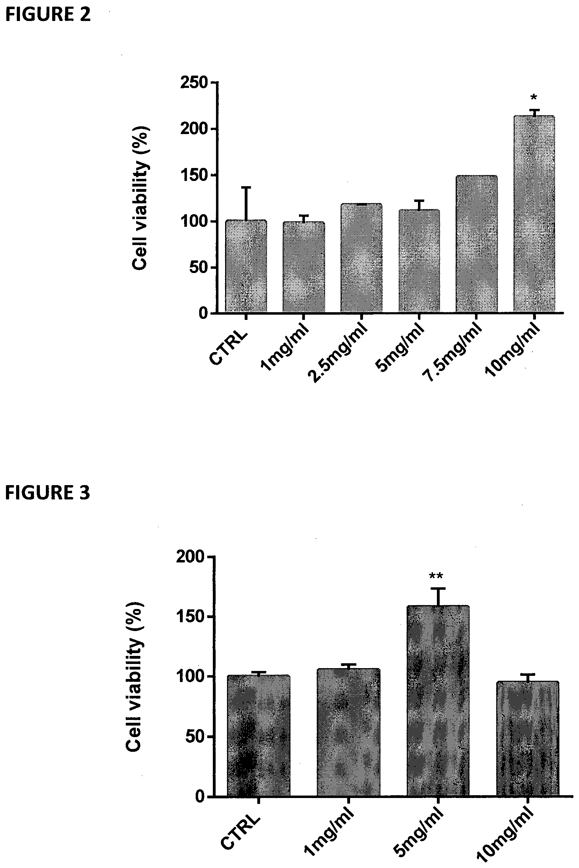

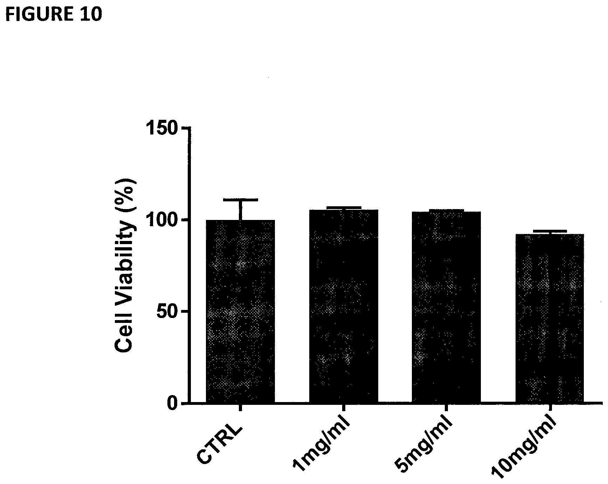

The composite gel obtained is subsequently put in contact on a substrate based on biomaterial 1 at different concentration s of hyaluronic acid for 24 hr or with DMEM not supplemented with hyaluronic acid (control). After this time the Alamar blue reagent was added to the samples and incubated at 37°C for 4 hr. The results are shown in figure 2 and expressed in terms of percentage of viability compared to the control. From figure 2 it can be observed that at all concentrations of biomaterial 1 the viability of the cells in the gel is higher than or equal to the control. In addition, it is possible to notice a substantial increase in viability as the concentration of hyaluronic acid increases. These results indicate that collagen, combined with low molecular weight hyaluronic acid in accordance with the present invention, has a consistent positive effect on cell viability; they also show that the separation between the two biopolymers within the biomaterial is a particularly effective solution in promoting cell viability. For reference purposes, the experiment was then repeated with the difference that the collagen composite gel obtained as above was deposited on a layer of biomaterial 2, containing in this case high molecular weight hyaluronic acid: the results, in the figure 10, show that using this hyaluronic acid, it is no longer observed the increase of viability compared to the control, observed in figure 2. Taken all together, figures 2 and 10 show that hyaluronic acid, even if separated from the biomaterial compartment containing the collagen, and particularly in this conformation, actively cooperates with collagen in promoting cell viability, provided that hyaluronic acid is at low molecular weight.

### Biomaterial 4 (System 3 not part of the invention)

The hUCMSCs were combined with the collagen and hyaluronic acid solution at pH 7.2 described in Example 1c and an interpenetrated gel of collagen and hyaluronic acid containing hUCMSCs has been obtained. The biomaterial 4 containing the cells is subsequently put in contact on a DMEM substrate and incubated for 24 hours. Biomaterial 3 is used as a control. After this time the Alamar blue reagent is added to the samples and incubated at 37°C for 4 hr. The results are shown in figure 3 and expressed in terms of viability percentage calculated as shown in Example 2.1. With this experiment it is possible to evaluate the effect of hyaluronic acid and collagen on viability when both are in contact with the cells. In figure 3, it can be observed that at all concentrations of hyaluronic acid in biomaterial 4 the viability of the cells in the gel is higher than or equal to the control, obtaining a statistically significant increase in viability ($p < 0.01$), at a specific concentration of 5mg/ml of hyaluronic acid.

## Example 4: Study of in vitro differentiation of hUCMSCs cells into type II alveolar cells

In order to evaluate the differentiation of hUCMSCs cells, in contact with biomaterials 1 to 4, in type II alveolar epithelial cells, the hUCMSCs cells were placed in contact with feeder cells able to simulate the physiological environment of niche/tissue of the lung, according to the experimental protocol reported in the literature [Ma N et al Cell Biol Int. 2011]. The MRC-5 cell line (ATCC CCL-171), derived from normal fetal lung tissue, has been used as feeder cells. In particular, the MRC-5 were grown in T-75 culture flasks (Falcon, Italy 156499), in the EMEM cell culture medium (Hyclone, USA) supplemented with 10% fetal bovine serum (FBS) and antibiotics (penicillin G 100 U/mL, streptomycin 100 $\mu$g/mL) and 2X nonessential amino acids, at 37°C at 5% $CO_2$. The cells were grown up to 80% of the confluence and were treated with 10 $\mu$g/ml of mitomycin C (Sigma n° 10107409001) in a basal medium for 2 hours at 37°C [Ma N et al Cell Biol Int 2011]. The feeder cells are then co-cultured in a precise 1:10 ratio with the hUCMSCs cells for differentiation studies.

To this aim, as culture medium to induce lung differentiation it was used SAGM (Lonza C-4124), a medium specifically used for the growth and maintenance of a mature distal pulmonary epithelium, which consists of basal medium plus the following factors: 0.5 mg/ml of BSA, 5 mg/ml of insulin, 10 mg/ml of transferrin, 30 mg/ml bovine pituitary extract, 0.5 mg/ml adrenaline (epinephrine), 6.5 ng/ml triiodothyronine, 0.1 ng/ml retinoic acid, 0.5 mg/ml cortisol, 0.5 ng/ml human EGF (epidermal growth factor) and antibiotics (gentamicin sulphate, 0.05 mg/ml; amphotericin-B, 0.05 mg/ml) supplemented with 5% fetal bovine serum (FBS).

To evaluate the differentiation of hUCMSCs cells, the qualitative and quantitative expression of surfactant protein C (SPC), a specific marker of differentiation in lung tissues, was evaluated by immunofluorescence technique [Ma N et al Cell Biol Int 2011]. SPC protein is the most important surfactant functional protein secreted in the alveoli. Indeed, the surfactant is synthesized and secreted by type II alveolar epithelial cells, also called pneumocytes, which differentiate between 24 and 34 weeks of gestation of the human fetus. It is composed of 70-80% of phospholipids (dipalmitoyl phosphatidylcholine-DPPC), about 10% of proteins (SP-A, SP-B, SP-C and SP-D) and 10% of neutral lipids, mainly cholesterol [Mertens et al Pulm Pharmacol Ther 2017]. In particular, SPC is a trans-membrane protein with a short extra-membrane domain expressed exclusively by type II alveolar cells after birth, it is stored in the lamellar bodies with the phospholipids of the surfactant until secretion into the alveolar space in which increases the stability and diffusion of phospholipids.

In particular, the differentiation of hUCMSCs into type II alveolar cells was evaluated under the following experimental conditions:

• Biomaterials 1 and 2 (System 1):

HUCMSCs cells were seeded in 35 mm fluorodish (World Precision Instruments, Inc) and cultured for 15 and 21 days on a layer of feeder cells as described above, using as culture medium biomaterials 1 and 2 containing SAGM and hyaluronic acid at the concentration of 5 mg/ml, prepared as described in Example 1a. Moreover, HUCMSCs cells cultured with DMEM, which does not induce differentiation, and cultured with SAGM alone, were used as controls.

• Biomaterial 4 (System 2):

HUCMSCs cells and feeder cells were seeded in the collagen solution at pH 7.2 described in Example 1b and an interpenetrated gel of collagen containing hUCMSCs and feeder cells has been obtained according to the described procedure. The cells in the gel were then placed in 35 mm fluorodish (World Precision Instruments, Inc) and cultured using, as a culture medium, biomaterial 1 containing SAGM and hyaluronic acid at 5 mg/ml concentration or DMEM and SAGM not containing hyaluronic acid (controls) for 15 and 21 days.

• Biometarial 4 (System 3, not part of the invention):

HUCMSCs cells and feeder cells were seeded in the collagen and hyaluronic acid solution at pH 7.2, as described in Example 1c, and an interpenetrated gel of collagen and hyaluronic acid containing hUCMSCs and feeder cells has been obtained according to the described procedure. The cells in the gel were then placed in 35 mm fluorodish (World Precision Instruments, Inc) and cultured using, , as a culture medium SAGM, biomaterial 1, at 5 mg/ml concentration or DMEM and SAGM not containing hyaluronic acid (controls) for 15 and 21 days.

All samples were fixed at 10% formalin (Sigma-Aldrich) for 1 h, the cells were permeabilized with 0.1% of Triton X-100, and blocking was carried out with 5% of serum albumin bovine BSA (Sigma). The samples were then incubated with an SPC rabbit antibody (Abcam AB40879) diluted to 1% BSA at 4°C overnight. After three washes with PBS, the secondary anti-rabbit antibody conjugated with green fluorophore "fitch" (Millipore, Billerica, MA, USA) was added to the cells for 3 hours at room temperature. Finally, the cell nuclei were stained with blue DAPI for 10 minutes at 37°C. The samples were observed by confocal microscope (Leica TCS SP8) with immersion and water objectives of 40X and 63X. The images were acquired with a resolution of 1024x1024 pixels.

The quantitative analysis of the confocal images was performed on five images for each sample after 21 days of incubation. The images acquired using a confocal microscope were analyzed using the ImageJ software "area calculator plugins" by calculating the percentage of the Mean Gray Value (MGV).

The data were reported as MGV normalized for the area of interest (ROI) of the individual cells.

Statistical analysis

In all histograms the results were expressed as mean $\pm$ standard deviation (SD). The repeated results were compared with the one-way analysis of variance (ANOVA) and a P value <0.05 was considered significant.

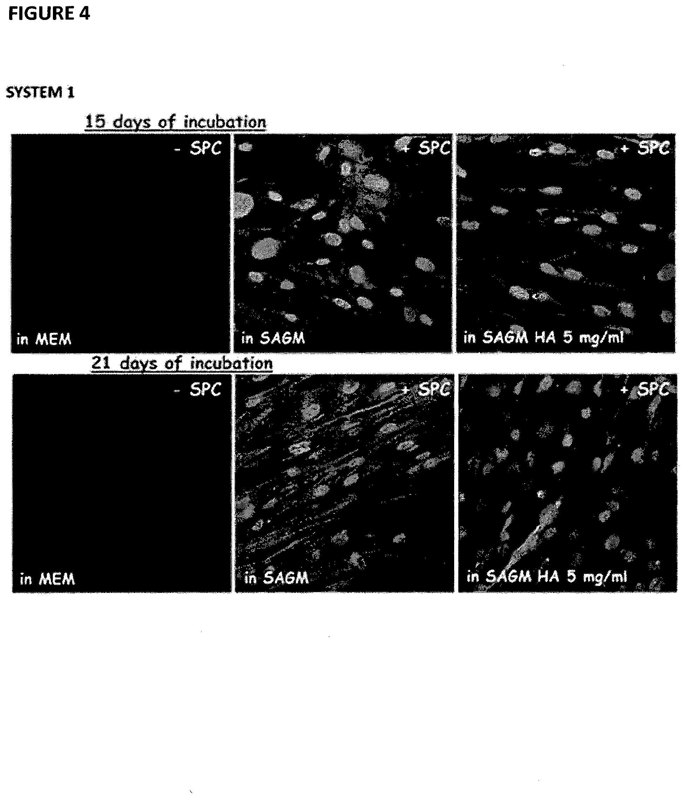

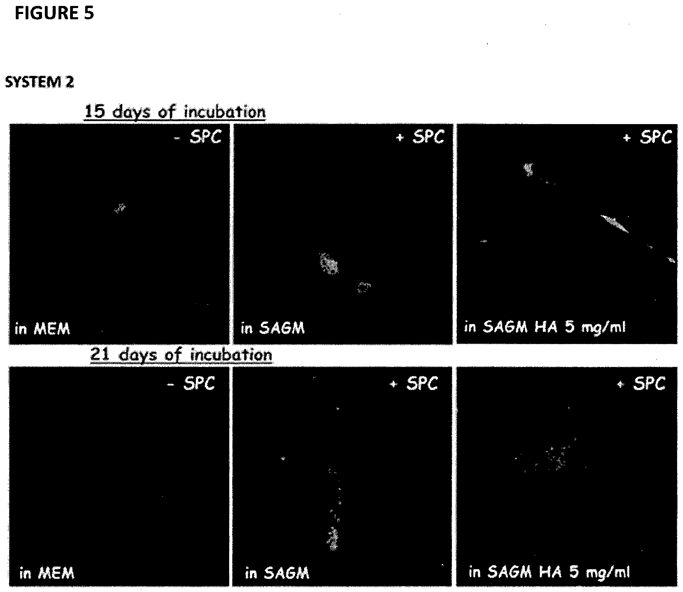

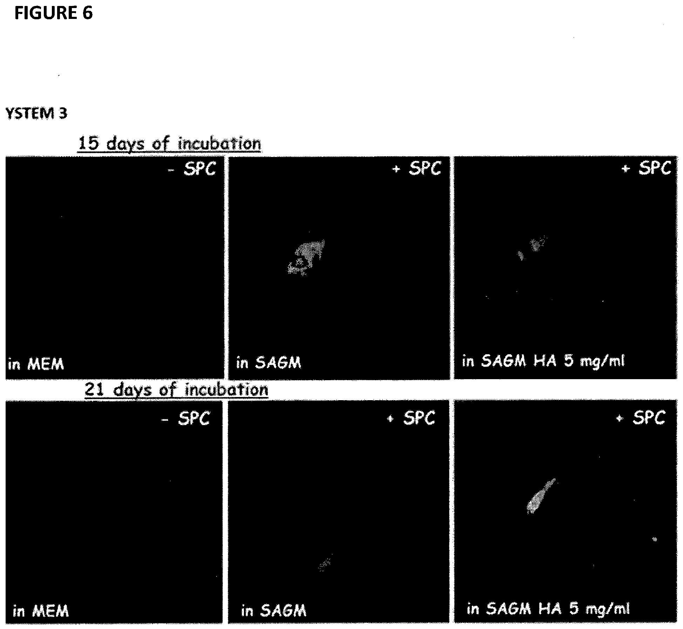

Confocal images (figures 4, 5 and 6), obtained by the immunofluorescence technique, show the qualitative expression of the specific marker SP-C in type II alveolar cells after culturing the cells according to System 1, 2 or 3, as described above for 15 and 21 days.

All the treated samples showed SPC antibody reactivity. Immunoreactive SPC cells have been observed clearly as fluorescence spots detected in a homogeneously diffused way at cytoplasmic level. Furthermore, in the figure 4 it is possible to observe, both at 15 and 21 days, that the differentiated cells have a morphology different from the known fibroblast-like morphology of the hUCMSCs, showing an approximately cuboidal shape with more regular dimensions, typical of epithelial cells.

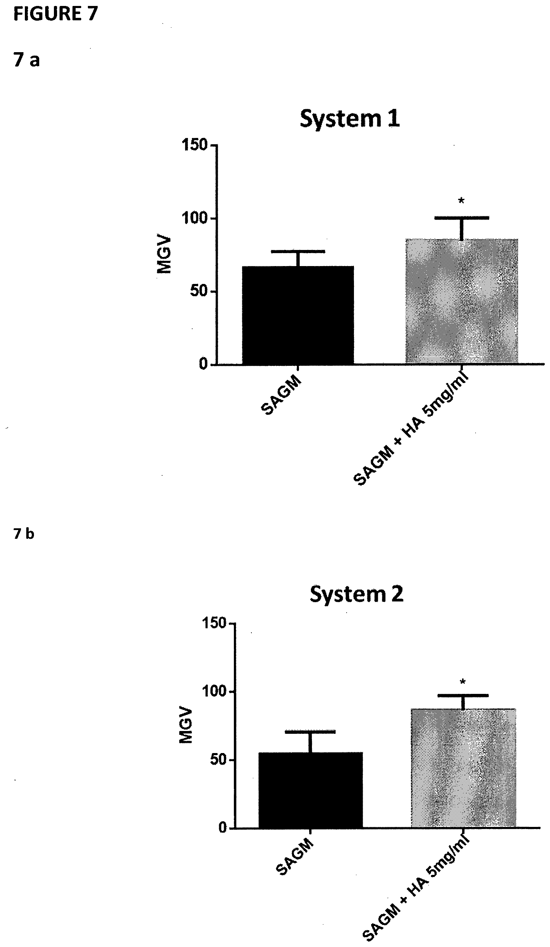

Figure 7 shows the quantitative analysis of the confocal images. The fluorescence intensity is expressed as mean gray value (MGV), in particular in figure 7 (a, b) the fluorescence intensities are reported for the hUCMSCs of System 1 and 2, respectively.

For both cells treated, according to System 1 and System 2, the fluorescence intensity, which indicates the presence of the surfactant protein C and therefore pneumocytic differentiation, is higher when the hUCMSCs are put in contact with the biomaterial 1 containing hyaluronic acid dissolved in SAGM, compared to cells put in contact with SAGM alone. It can be noted that quantitatively the cells are able to differentiate in both Systems 1 and 2, with higher differentiation in the case of System 2.

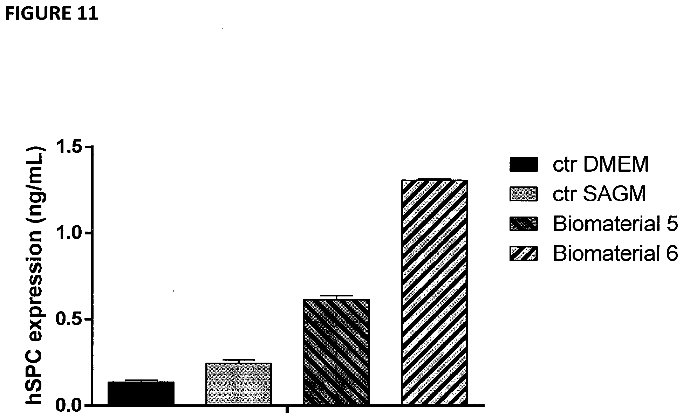

In order to evaluate the effects of the addition of secretome in the present biomaterials, two further biomaterials 5 and 6 of equal composition (2.4 mg/ml of collagen and 5 mg/ml of hyaluronic acid with a molecular weight of 200 KDa) were made, respectively without or with lyophilized secretome, at a concentration of 0.2 mg/ml (in an amount equivalent to $2*10^4$ human adipose mesenchymal cells). The biomaterials were made in accordance with System 3, with a first phase containing collagen, hyaluronic acid, mesenchymal cells, deposited on a second phase containing DMEM (non-differentiated control), or SGAM nutrient for differentiation (differentiated control), or additional low molecular hyaluronic acid weight without secretome (biomaterial 5), or additional low molecular weight hyaluronic acid with secretome (biomaterial 6). The results are illustrated in figure 11. It possible to observe, that both biomaterials 5 and 6 according to the invention, obtain an increase in differentiation compared to controls; moreover, the addition of secretome in biomaterial 6 has greatly amplified the amount of differentiation obtained by the biomaterial, compared to biomaterial 5, without secretome.

In summary, these experiments show that hyaluronic acid, when used in the present range of low molecular weight, is effective in promoting the viability of mesenchymal cells and this effect is amplified in the presence of collagen, in particular when collagen is present in biomaterial as a phase separated from hyaluronic acid. The hyaluronic acid at low molecular weight also promotes the effect of cellular signals aimed to the differentiation in the pneumocytic sense with a higher effect compared to the use of the SAGM conditioned medium alone and moreover the addition of collagen further promotes this differentiation; and a further improvement of differentiation is achieved by incorporating secretome.

### Example 5: Rheological properties of the analyzed biomaterials

In order to evaluate the rheological properties of the used biomaterials, the analysis of viscoelastic properties was carried out by a stress - controlled rotational rheometer (Mars III, Rheometer HAAKE, Waltham, MA USA), using a parallel plate geometry at the controlled temperature of 20 and 37°C. The samples were subjected to oscillatory shear tests with a variable frequency between 0.01 and 10 Hz. In order to identify the linear viscoelastic response range of the materials, preliminary amplitude sweep tests were performed on the samples at the oscillation frequency of 1 Hz. The tests were repeated at least three times on each sample.

The dependence of the elastic modulus G' and the viscous modulus G" as a function of frequency, the so-called "mechanical spectrum" has been evaluated for the biomaterials of the present invention.

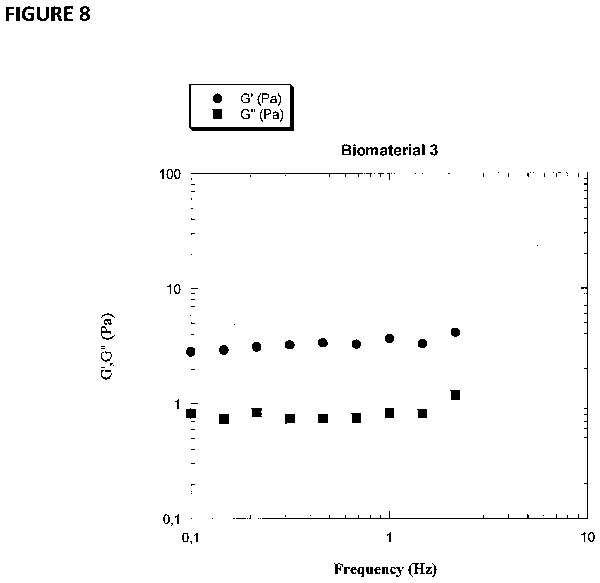

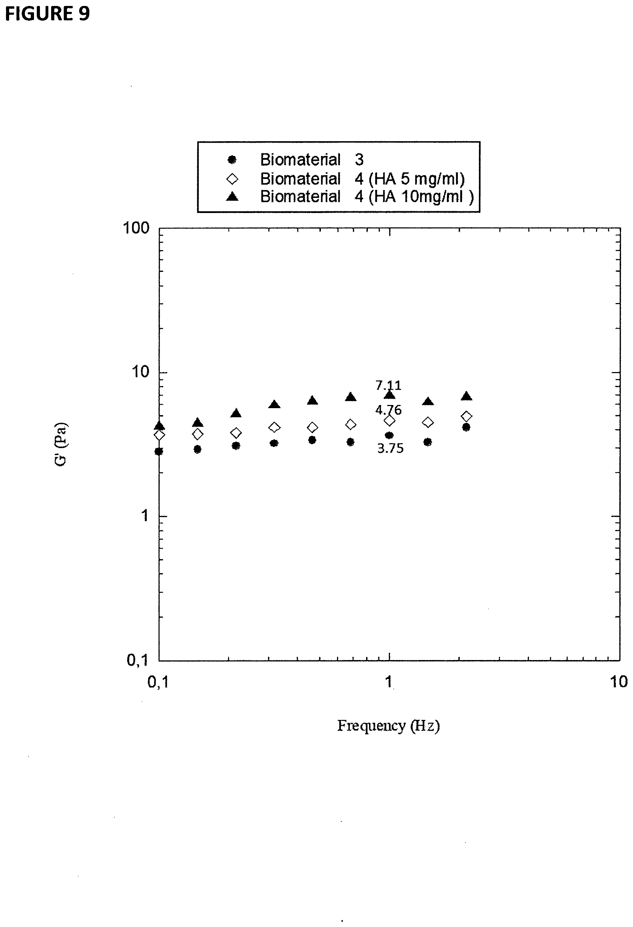

Figure 8 shows, as an example, the mechanical spectrum of biomaterial 3. As can be seen in figure 8, the elastic modulus G' is little dependent on the frequency and it is greater than the viscous modulus G" in the whole frequency range analysed, this behaviour is typical of "gels" and it is characteristic of soft tissues such as cartilage, nucleus pulposus of the intervertebral disc, etc. Figure 9 shows the dependence of the viscous modulus on the frequency of biomaterial 4 at two different concentrations of hyaluronic acid: 5 mg/ml and 10 mg/ml. As it can be seen from figure 9, at the frequency of 1 Hz, the value of the elastic modulus is 3.75 for biomaterial 3 and 4.7 and 7.11 for biomaterial 4 at 5 and 10 mg/ml concentrations of hyaluronic acid respectively. This indicates that the presence of hyaluronic acid leads to an increase in the elastic properties of the network constituting the biomaterial, stabilizing its structure.

## Claims

1. Biomaterial comprising:

   (a) hyaluronic acid or a salt thereof, having a molecular weight between 100 and 500 KDa;
   (b) collagen;
   (c) human mesenchymal cells and/or secretome thereof;

   said biomaterial containing two phases, wherein at least part of component (b) is present as a separate phase from component (a), said separate phase also containing the component (c).

2. Biomaterial according to claim 1, wherein said hyaluronic acid or a salt thereof has a molecular weight between 100 and 300 KDa, more preferably between 160 and 240 KDa.

3. Biomaterial according to claims 1 to 2 wherein said collagen is type I or type III collagen, even more preferably type I collagen.

4. Biomaterial according to claims 1 to 3, in the form of a gel or a viscous solution, with a dynamic viscosity between 0.001 and 100 Pa s, preferably between 0.004 and 10 Pa s, measured at a temperature of 25°C in a steady-state shear flow at shear rate equal to 10 sec$^{-1}$.

5. Biomaterial according to claims 1 to 4, for use in therapy.

6. Biomaterial for use according to claim 5, for use in the treatment of pulmonary diseases.

7. Biomaterial for use according to claim 6, wherein said pulmonary pathologies are chronic obstructive pulmonary disease (COPD), asthma, idiopathic fibrosis (IPF) and pulmonary bronchodysplasia (BPD) of the premature infant.

## Patentansprüche

1. Biomaterial, umfassend:

   (a) Hyaluronsäure oder ein Salz davon, mit einem Molekulargewicht zwischen 100 und 500 KDa;
   (b) Kollagen;
   (c) humane mesenchymale Zellen und/oder deren Sekretom;

   wobei das Biomaterial zwei Phasen enthält, wobei wenigstens ein Teil von Komponente (b) als eine von Komponente (a) separate Phase vorliegt, wobei diese separate Phase auch die Komponente (c) enthält.

2. Biomaterial nach Anspruch 1, bei dem die Hyaluronsäure oder ein Salz davon ein Molekulargewicht zwischen 100 und 300 KDa, noch mehr bevorzugt zwischen 160 und 240 KDa, aufweist.

3. Biomaterial nach den Ansprüchen 1 bis 2, bei dem das Kollagen ein Typ I oder Typ III Kollagen, noch mehr bevorzugt, ein Typ I Kollagen ist.

**4.** Biomaterial nach den Ansprüchen 1 bis 3, in Form eines Gels oder einer viskosen Lösung, mit einer dynamischen Viskosität zwischen 0,001 und 100 Pa s, vorzugsweise zwischen 0,004 und 10 Pa s, gemessen bei einer Temperatur von 25°C in einer stationären Scherströmung mit einer Schergeschwindigkeit, die gleich 10 s$^{-1}$ ist.

**5.** Biomaterial nach den Ansprüchen 1 bis 4, zur Verwendung in der Therapie.

**6.** Biomaterial zur Verwendung nach Anspruch 5, zur Behandlung von Lungenerkrankungen.

**7.** Biomaterial zur Verwendung gemäß Anspruch 6, wobei die Lungenerkrankungen chronisch-obstruktive Lungenerkrankung (COPD), Asthma, idiopathische Lungenfibrose (IPF) und bronchopulmonale Dysplasie des frühgeborenen Kindes sind.

**Revendications**

**1.** Biomatériau comprenant :

(a) de l'acide hyaluronique ou un de ses sels, ayant un poids moléculaire compris entre 100 et 500 KDa ;
(b) du collagène ;
(c) des cellules mésenchymateuses humaines et/ou leur sécrétome ;

ledit biomatériau contenant deux phases, dans lesquelles au moins une partie du composant (b) est présente en tant que phase séparée du composant (a), ladite phase séparée contenant également le composant (c).

**2.** Biomatériau selon la revendication 1, dans lequel ledit acide hyaluronique ou un de ses sels a un poids moléculaire compris entre 100 et 300 KDa, plus préférentiellement entre 160 et 240 KDa.

**3.** Biomatériau selon les revendications 1 à 2, dans lequel ledit collagène est de type I ou de type III, de préférence de type I.

**4.** Biomatériau selon les revendications 1 à 3, sous forme de gel ou de solution visqueuse, avec une viscosité dynamique comprise entre 0,001 et 100 Pa s, de préférence entre 0,004 et 10 Pa s, mesurée à une température de 25°C dans un flux de cisaillement en régime permanent à un taux de cisaillement égal à 10 sec-1.

**5.** Biomatériau selon les revendications 1 à 4, pour une utilisation en thérapie.

**6.** Biomatériau à utiliser selon la revendication 5, pour une utilisation dans le traitement des maladies pulmonaires.

**7.** Biomatériau pour une utilisation selon la revendication 6, dans lequel lesdites pathologies pulmonaires sont la bronchopneumopathie chronique obstructive (BPCO), l'asthme, la fibrose idiopathique (IPF) et la dysplasie bronchopulmonaire (BPD) du nourrisson prématuré.

**FIGURE 1**

**1 a**

**1 b**

**FIGURE 2**

**FIGURE 3**

# FIGURE 4

**SYSTEM 1**

15 days of incubation

21 days of incubation

**FIGURE 5**

SYSTEM 2

15 days of incubation

21 days of incubation

**FIGURE 6**

YSTEM 3

15 days of incubation

| − SPC | + SPC | + SPC |
|---|---|---|
| in MEM | in SAGM | in SAGM HA 5 mg/ml |

21 days of incubation

| − SPC | + SPC | + SPC |
|---|---|---|
| in MEM | in SAGM | in SAGM HA 5 mg/ml |

**FIGURE 7**

7 a

System 1

7 b

System 2

**FIGURE 8**

**FIGURE 9**

**FIGURE 10**

**FIGURE 11**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5633003 A, Cantor
- US 20080311088 A, Chang
- US 20100266552 A, Jenkin
- US 20090274665 A, Akabutu
- WO 2017164467 A1
- WO 2018085516 A2
- US 20140227235 A1
- WO 03015802 A1

- WO 2015073786 A1
- WO 2011047345 A2
- EP 2554176 A1
- WO 2014127232 A2
- WO 2015023720 A1
- WO 2010114319 A2
- IT 156499

**Non-patent literature cited in the description**

- **POSTMA et al.** *The New Engl Jour of med*, 2015
- **SLATS et al.** *Therap advan in resp disease*, 2016
- **GOHY et al.** *J of the British Society for Aller and Clin Immuno*, 2016
- **MERTENS et al.** *Pulm Pharmacol Ther*, 2017
- **BHANDARI et al.** *Pediatrics*, 2009
- **CHAO CM et al.** *Front Med*, 2015
- **PIERRO et al.** *Thorax*, 2013
- **BEERS et al.** *J Clin Invest*, 2016
- **PIERRO M ; THÉBAUD B et al.** *Thorax*, 2013
- **LAUBE et al.** *Int J Biochem Cell Biol*, 2016
- **CHANG YS et al.** *J Pediatr*, 2014

- **NITKIN CR et al.** *Stem Cells Transl Med*, 2017
- **O'REILLY, THÉBAUD et al.** *CellTissue Res*, 2017
- *Life Sciences*, 2004, vol. 75 (26), 3087-3102
- **PITTENGER et al.** *Science*, 1999
- **BAKSH et al.** *Stem Cells*, 2007
- **COLLINS JJP et al.** *Front Med (Lausanne)*, 2017
- **COLLINS, THEBAUD et al.** *Birth Defects Research (part A)*, 2014
- **HAN YF et al.** *Cytotechnology*, 2013
- **SMITH JR et al.** *Curr Protoc Stem Cell Biol*, 2017
- **MA N et al.** *Cell Biol Int.*, 2011
- **MA N et al.** *Cell Biol Int*, 2011